# EUROPEAN PATENT APPLICATION

(11) **EP 4 837 527 A1**
(43) Date of publication of application: **11.11.2026**
(21) Application number: 25174181.5
(22) Date of filing: 05.05.2025
(51) Int. Cl.: A61B 17/32, A47J 43/25, B02C 19/20, A61B 17/322, G01N 1/28

(54) **A GRINDER, ESPECIALLY FOR PREPARING MICROGRAFTS FROM SOFT TISSUE SUCH AS CARDIAC TISSUE**

(71) Applicant: Epiheart Oy, 02200 Espoo (FI)
(72) Inventor: Kuuva, Aleksi, 00520 Helsinki (FI); Kronström, Kai, 02200 Espoo (FI)
(74) Representative: Väisänen, Olli Jaakko

(57) **Abstract**

To improve cutting properties of a cutting blade (2) in a grinder (1, 100; 20), especially for preparing micrografts from soft tissue such as cardiac tissue, it is suggested a grinder (1, 100; 20) that comprises:
at least one grinding element (1, 100) comprising a number of cutting blades (2), each of which has been made by bending, to an angle (α), a region partially separated from the grinding element (1, 100) by a number of through cuts (4) made in the grinding element (1, 100), whereby the bending has been made at the non-separated part between the cutting blade (2) and the grinding element (1, 100) adjacent to a surrounding opening made by the number of through cuts (4).
Each of the number of cutting blades (2) has at least one beveled face (3), and the surrounding opening in the grinding element (1, 100) has respectively at least one beveled face (6) and wherein, through the bending, the at least one beveled face (3) of the cutting blade and the at least one beveled face (6) in the surrounding grinding element (1, 100) are in mutual contact.

## Description

### Field of the invention

The invention relates for grinders, especially for preparing micrografts from soft tissue such as cardiac tissue.

### Background art

A recent advance in functional cardiac repair is the utilization of an individual's autologous, processed cardiac micrografts derived from atrial appendages. Micrograft transplants produced from cardiac atrial appendages comprehensively harbor most myocardial cell types intricately meshed with their supporting extracellular matrix. Their targeted delivery is achievable upon open heart surgery by epicardial transplantation, and they are apt targets for gene therapy applications. This is explained in more detail in Kankuri, E., Karjalainen, P., Vento, A. (2023). Atrial Appendage-Derived Cardiac Micrografts: An Emerging Cellular Therapy for Heart Failure. In: Haider, K.H. (eds) Cardiovascular Applications of Stem Cells. Springer, Singapore. https://doi.org/10.1007/978-981-99-0722-9_7 Atrial Appendage-Derived Cardiac Micrografts: An Emerging Cellular Therapy for Heart Failure. (DOI https://doi.org/10.1007/978-981-99-0722-9 7)

For preparing micrograft transplants, a grinder is necessary. The present applicant has developed an apparatus and method for mechanically disintegrating soft tissues into micrografts, and a feeder piece used in connection with the disintegration of tissue. These are described in published European patent 4231941. Generally, soft tissues include fat, muscle, nerves, blood vessels, skin, cartilage, ligaments, tendons, and other fibrous tissues. The previously developed grinder is suitable for preparing micrograft transplants.

### Objective of the invention

The objective of the present invention is, on one hand, to improve dynamical grinding properties of a grinder, especially for preparing one suitable for preparing micrografts from soft tissue such as cardiac tissue. On the other hand, the objective of the present invention is to simplify manufacturing of such a grinder such that the grinder may be capable of producing more useful or consistent cardiac tissue micrografts.

Both objectives can be met with the grinder according to claim 1.

The dependent claims describe various advantageous aspects of the grinder.

### Advantages of the invention

A grinder, especially for preparing micrografts from soft tissue such as cardiac tissue, comprises at least one grinding element comprising a number of cutting blades, each of which has been made by bending, to an angle (α), a region partially separated from the grinding element by a number of through cuts made in the grinding element, whereby the bending has been made at the non-separated part between the cutting blade and the grinding element adjacent to a surrounding opening made by the number of through cuts.

Each of the number of cutting blades has at least one beveled face, and the surrounding opening in the grinding element has respectively at least one beveled face. Through the bending, the at least one beveled face of the cutting blade and the at least one beveled face in the surrounding grinding element are in mutual contact.

The inventors have observed that a grinder with a number of such cutting blades performs better than the grinders they have used before. In particular, it may increase the possibility to obtain a clean cut. Without be willing to be bound by theory, a research hypothesis is that the improvement may result from the cutting action causing further bending of the blade, which, because of the mutual contact, will pre-tension the cutting blade and in some manner increase the stiffness of the cutting blade or in some other manner facilitate counteracting the cutting blade to penetrate in soft tissue (especially cardiac tissue) and cut it.

An advantage is that the manner of implementation of the number of cutting blades may enable manufacturing the grinding element of thinner material (as base material) which contributes to the sharpness of the cutting blades or clean cutting capability of the cutting blades, while still ensuring that the cutting blades and the grinding element have a sufficient strength to resist the deforming forces caused by the tissue that is being processed by the grinder. The sharpness may be increased by use of a thinner material with considerably less sharpening.

Preferably, when the grinder is used in grinding, upon a force that will be exerted to a cutting blade by material to be grinded causing a torque trying to bend the blade further and resulting in the beveled faces in mutual contact to cause a counterforce which will be directed towards the grinding element adjacent the surrounding opening, effecting a bending type tensioning of the cutting blades. The inventors have observed that this kind of configuration may provide the grinder having a number of such cutting blades with a particularly good cutting performance in soft tissue (especially cardiac tissue).

Preferably, each of the number of cutting blades has beveled faces at opposite sides, each beveled face, through the bending, being in mutual contact with a respective beveled face in the grinding element adjacent the surrounding opening, such that the bending-type tensioning of the cutting blade will be directed to deform the cutting blade into a convex shape. The inventors have observed that this kind of configuration may provide the grinder having a number of such cutting blades with a particularly good cutting performance in soft tissue (especially cardiac tissue).

Preferably, the through cuts are beveled through cuts having V shape, such that the point of the V shape defines an obtuse angle, a straight angle or an acute angle in the grinding element. This kind of configuration simplifies the sharpening process during manufacturing of the grinder. The point of the V shape may not need sharpening at all.

Advantageously, the sides of the V shape may be beveled such that the beveled edges define at least one acute angle between the surface of the grinding element before bending, in other words such that the at least one, preferably both, of the beveled edges interact with each other and limit the bending of the cutting blade and transfer a torque exerted on it to the grinding element via one or both of the beveled edges to tensioning of the cutting blade. With this kind of configuration of the grinder, the height of the cutting blades (which have been bent from a base blate) can be kept sufficiently constant - in other words, avoiding further bending to a larger angle which would impact the height of the cutting blade may be better prevented. This effect may be utilized to provide better-quality grinding results, especially cardiac micrografts.

Advantageously, the beveled through-cuts may intersect such that the beveled edges define the V shape which is part of a pointed tip. This simplifies the sharpening process even further during manufacturing of the grinder, since in addition to the point of the V shape which may not need sharpening at all, also the bevels manufactured by cutting may already have a sufficient cutting effect. In this situation, it eventually may suffice to sharpen and/or flatten the cutting blade from top.

Preferably, the cutting blades may be arranged at directional groups, each of which has an opposite cutting direction. This enables using other than rotatory sample carrier in the grinder so that the grinder may be implemented in a simpler manner. In this kind of grinder, the matter that is processed with the grinder can be moved in a reciprocal manner, such as back and forth. The inventors have observed that cutting blades in opposite direction will improve the grinding result and grinding speed. Both improvements may be beneficial for the treatment success with cardiac micrografts, for example.

Some or all of the number of cutting blades may have been treated preferably mechanically such as by grinding, sanding and/or polishing, from the bended side which protrudes from the grinder element such that the cutting blade has a sharpened and/or flattened region from the top that is preferably parallel or essentially parallel with a section of the grinding element adjacent to the surrounding opening. In this manner, the soft tissue piercing and cutting capability of the cutting blades may be improved.

Preferably, the grinding element is or comprises a plate-like element, from which the cutting blades protrude. In an alternative embodiment, the grinding element may be or comprise a cylinder, from which the number of cutting blades protrudes.

When the grinding element is or comprises the plate-like element, the grinder preferably further comprises a sled-like sample carrier, a handle arranged on the sled such that a movement of the handle moves the sled in relation to the grinding element, the sled arranged in a housing that comprises the grinding element and limits the movement of the sled however allowing a reciprocal such as back-and-forth motion, and pressing means for pushing material received via a material receiving opening such that the cutting blades will cut the material responsive to the movement of the handle. This configuration may be used to simplify the manufacture of the grinder, especially in contrast to the grinders in which rotation is used to grind soft tissue samples.

The grinder may further comprise at least two skids arranged to keep the part of sled, which is located between the skids, in an elevated position with respect to the grinding element such that the elevated position has a clearance which is above the bent cutting blades, and wherein, preferably, the housing is configured to limit the movement of the sled such that each of the skids is moveable on the grinding element only at a region that is free of said cutting blades. In this manner, it can be ensured that the cutting blade will not engage with the sled that is used as sample carrier.

The height of the skids is preferably selected such that the cutting blades do not come into contact with the sled.

There may be a coding in the sled and/or in the housing, defining in which direction the sled is placeable into the grinder, allowing the grinding element to be replaced or cleaned.

Advantageously, the through cuts have been made using laser cutting or plasma cutting. With these cutting methods, a sufficiently sharp edge can be manufactured such that no or even minimal post-cutting treatment such as polishing will be necessary.

Preferably, the grinding element (and also for the base plate) comprises or is made of stainless steel, especially austenitic stainless steel that comprises iron, chromium (between 16-18%), nickel (10-12%) and molybdenum (2-3%), up to 2% manganese, with possibly small (<1%) quantities of silicon, phosphorus and sulfur possibly also being present. The inventors have found out that a particularly suitable material is stainless spring steel, especially SAE 316L.

The thickness of the grinding element (and also of the base plate) is advantageously between 0,3 mm - 0,6 mm, and most advantageously 0,4 mm, especially for the SAE 316L grade steel. This particular configuration appears to provide a superior sharpness and durability, while ensuring relatively constant manufacturing.

### List of drawings

In the following, the invention is described in more detail with reference to the exemplary embodiments shown in the attached drawings, of which:
FIG 1 - 13 illustrate the manufacturing of a grinding element which shows an exemplary manufacture of a cutting blade. More specifically,
I cutting
   FIG 1 (view from top) and FIG 3 (perspective view) illustrate a base in which a base blade has been partially separated from the grinding element (base) by two intersecting through cuts made in the grinding element; and
   FIG 2 illustrates section II-II.
II bending
   FIG 4 (view from top), FIG 6 (perspective view), FIG 7 (view from front) and FIG 8 (view from side) illustrate the bending of the base blade of FIG 1, 3 whereby the bending has been made at the non-separated part between the base blade and the grinding element (base) adjacent to a surrounding opening made by the two through cuts; and
   FIG 5 illustrates section V-V.
III sharpening/flattening
   FIG 9 (view from top), FIG 11 (perspective view), FIG 12 (view from front) and FIG 13 (view from side) illustrate sharpening or flattening of the bent base blade of FIG 4, 6, 7, 8; and
   FIG 10 illustrates section X-X.
IV method of using sharpened/flattened base blades in grinder
   FIG 14 (first perspective view), FIG 15 (top view) and FIG 16 (second perspective view) show grinders containing a flat-like grinding element comprising a number of cutting blades.

Same reference numerals refer to same technical parts in all FIG.

### Detailed description

### I cutting

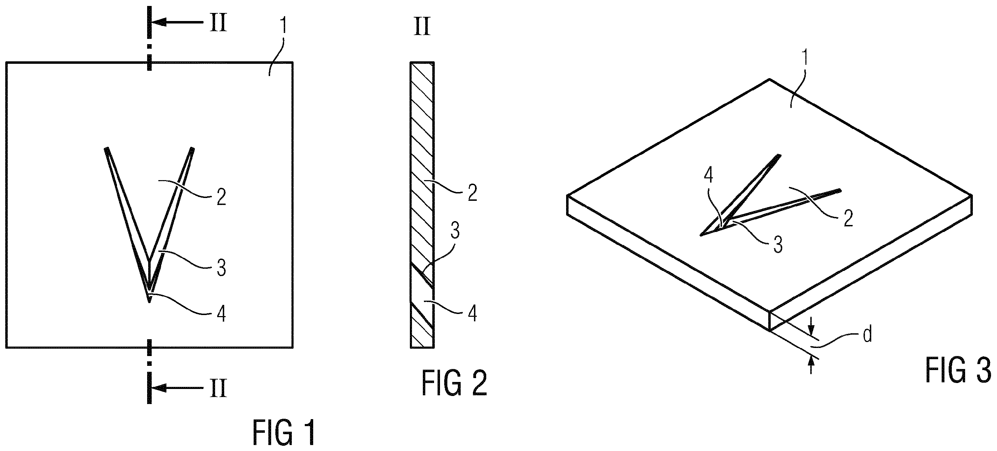

FIG 1 (view from top) and FIG 3 (perspective view) illustrate a base 1 in which a base blade 2 has been partially separated from the grinding element (base 1) by two intersecting through cuts 4 made in the grinding element.

As can be seen, the through cuts 4 have been made not straight but in an inclined angle to the base blade. This results the cutting blade 2 to have beveled faces 3. But in this manner, also the surrounding opening in the base has respectively beveled faces 6. This can be seen in FIG 2 which illustrates section II-II.

The example in FIG 1 - 3 has been realized with two through-cuts that are intersecting. In principle, instead of two, the number of through-cuts could be more (such as, three, four, five, ...).

### II bending

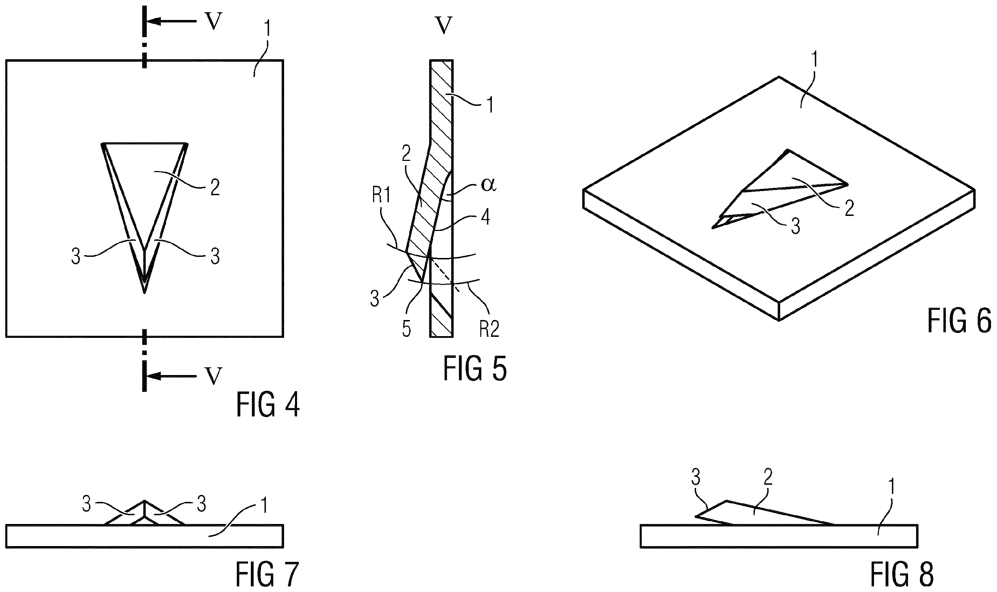

FIG 4 (view from top), FIG 6 (perspective view), FIG 7 (view from front) and FIG 8 (view from side) illustrate the bending of the base blade 2 of FIG 1, 3 whereby the bending has been made at the non-separated part between the base blade 2 and the grinding element (base 1) adjacent to a surrounding opening made by the two through cuts.

Through the bending, the beveled faces 3 of the base blade 2 and the beveled faces 6 in the surrounding grinding element (base 1) will be in mutual contact. This can be understood by viewing FIG 4 and FIG 6. FIG 5 illustrates section V-V. R1, R2 illustrate distances from bending line.

### III sharpening/flattening

At least in theory, and depending on the application, the base blade 2 may be used without flattening sharpening and it may thus form the cutting blade 2. But in the preferred embodiment, the cutting blade 2 is sharpened or flattened from the top.

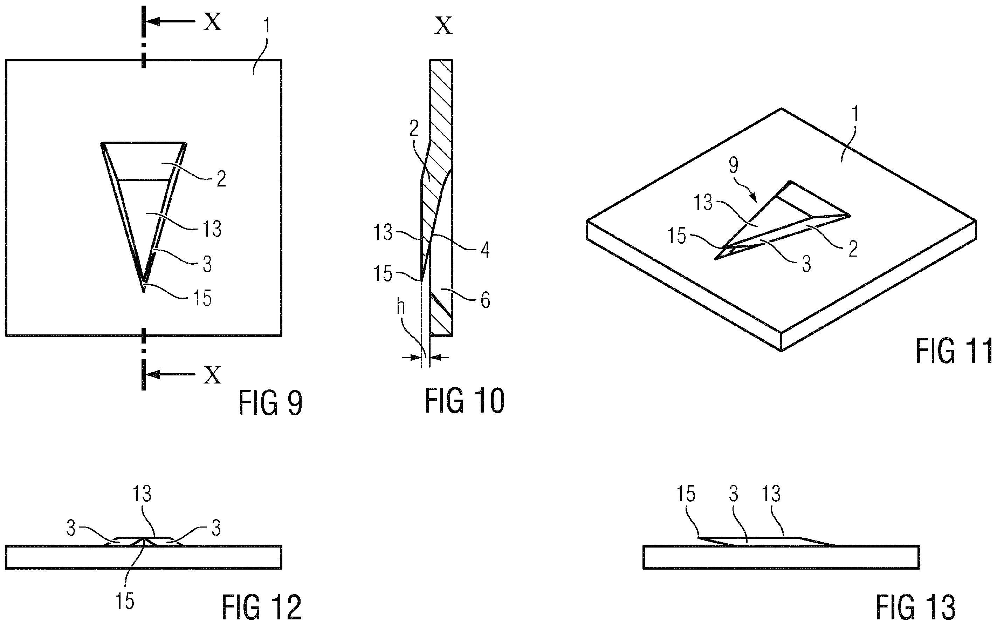

FIG 9 (view from top), FIG 11 (perspective view), FIG 12 (view from front) and FIG 13 (view from side) illustrate sharpening or flattening of the bent base blade 2 of FIG 4, 6, 7, 8. FIG 10 illustrates section X-X.

As can be seen, especially in FIG 10, the cutting blade 2 has been treated -preferably mechanically, such as by grinding, sanding and/or polishing- from the bended side which protrudes from the grinder element (base 1) such that the cutting blade 2 has a sharpened and/or flattened region 13 from the top that is preferably parallel or essentially parallel with a section of the grinding element (base 1) adjacent to the surrounding opening.

In this manner, the pointed tip 15 may be made very sharp with relatively little effort.

Further, the height h of the cutting blade 2 can be defined precisely. This may improve the homogeneity of the samples prepared with the grinder, and thus in a way contribute to increased normalizability of the treatment expectations of epicardial transplantation.

### IV method of using sharpened/flattened base blades in grinder

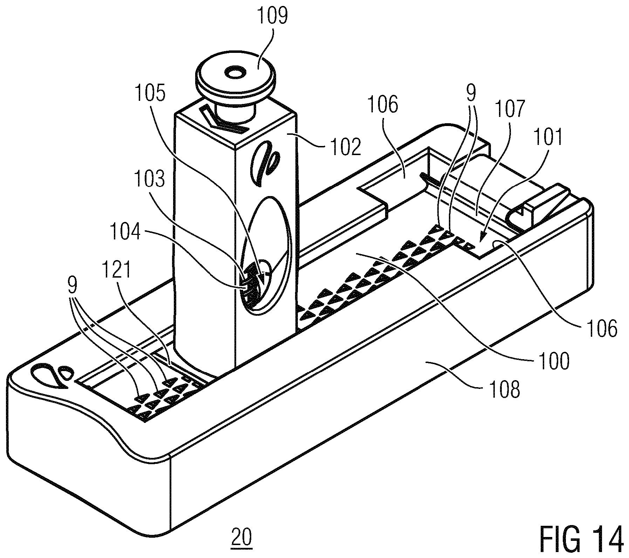

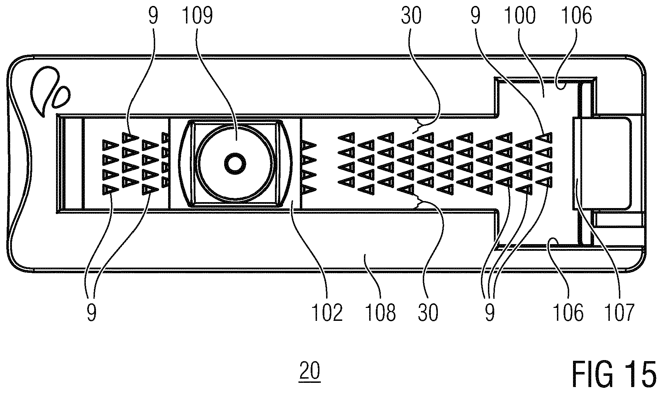

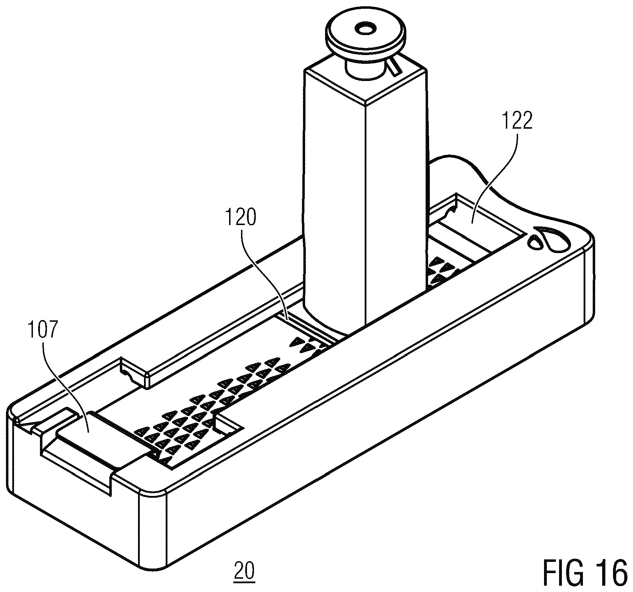

FIG 14 (first perspective view), FIG 15 (top view) and FIG 16 (second perspective view) show a grinder 20 containing a flat-like grinding element 100 which comprises a number of cutting blades 2 which protrude from the grinding element 100 and preferably are flattened and/or sharpened.

The grinder 20 comprises a sled-like sample carrier 121, a handle 102 arranged on the sled 121 such that a movement of the handle 102 moves the sled 121 in relation to the grinding element 100.

The sled 121 is preferably arranged in a housing 108 that comprises the grinding element 1, 100 and limits the movement of the sled 121 however allowing a reciprocal such as back-and-forth motion, and pressing means (such as, rod 104, and loading spring 103) for pushing material received via a material receiving opening 105 such that the cutting blades 2 will cut the material responsive to the movement of the sled-like sample carrier 121.

The grinder 20 further comprises a number, such as two, skids (not shown in FIG 14-16) arranged to keep the part of sled 121 which is located between the skids, in an elevated position with respect to the grinding element 100 (more precisely, with respect to the cutting blades 2), such that the elevated position has a clearance D (D > h) which is above the bent cutting blades 2, and wherein, preferably, the housing 108 is configured to limit the movement of the sled 121 such that each of the skids is moveable on the grinding element 100 only at a region 30 that is free of the cutting blades 2.

The height of the skids is selected such that the cutting blades 2 do not come into contact with the sled 121 i.e. the height of the skids > h.

There may be a coding in the sled 121 and/or in the housing 108, defining in which direction the sled 121 is placeable into the grinder 20, allowing the grinding element 100 to be replaced or cleaned.

### V Some aspects of the grinders (this relates to preferred embodiments: base plate 1, plate 100, cylinder embodiment, and the grinder 20)

The base plate 1, the grinding element 100 and the grinder 20 may all be designated as "grinder". Such a grinder (especially for preparing micrografts from soft tissue such as cardiac tissue) comprises:
at least a grinding element (base 1, grinding element 100) comprising a number of cutting blades 2, which have been made by bending, to an angle α, at least one region partially separated from the grinding element by a number of through cuts 4 made in the grinding element. The bending has been made at the non-separated part between the cutting blade 2 and the grinding element adjacent to a surrounding opening made by the number of through cuts 4.

The number of cutting blades 2 have at least one beveled face 3, and the surrounding opening in the grinding element has respectively at least one beveled face 6. Through the bending, the at least one beveled face 3 of the cutting blade and the at least one beveled face 6 in the surrounding grinding element 1, 100 are in mutual contact.

When the grinder is used in grinding, upon a force that will be exerted to the number of cutting blades 2 by material to be grinded causing a torque trying to bend the blade 2 further and resulting in the beveled faces 3, 6 in mutual contact to cause a counterforce at the at least one beveled face 3, a counterforce may be directed towards the grinding element adjacent the surrounding opening, effecting a bending type tensioning of the cutting blades 2.

The number of cutting blades 2 may have beveled faces 3 at opposite sides, each beveled face 3, through the bending, being in mutual contact with a respective beveled face 6 in the grinding element adjacent the surrounding opening, such that the bending-type tensioning of the cutting blade will be directed to deform the cutting blade 2 into a convex shape.

The through cuts 4 may be beveled through cuts having V shape, such that the point of the V shape defines an obtuse angle, a straight angle or an acute angle in the grinding element.

The sides of the V shape may be beveled such that the beveled edges 3 define at least one acute angle between the surface of the grinding element before bending, in other words such that the at least one, preferably both, of the beveled edges 3 interact with each other and limit the bending of the cutting blade 2 and transfer a torque exerted on it to the grinding element via one or both of the beveled edges 3 to tensioning of the cutting blade 2.

The beveled through-cuts 4 may intersect such that the beveled edges 3 define the V shape which is part of a pointed tip 15.

The cutting blades 2 may be arranged at directional groups each of which has an opposite cutting direction.

Some or all of the number of cutting blades 2 may have been treated preferably mechanically, such as by grinding, sanding and/or polishing, from the bended side which protrudes from the grinder element such that the cutting blade 2 has a sharpened and/or flattened region 13 from the top that is preferably parallel or essentially parallel with a section of the grinding element adjacent to the surrounding opening.

The grinding element may be or comprise a cylinder, from which the number of cutting blades 2 protrudes.

Alternatively, the grinding element may be or comprises a plate-like element, from which the cutting blades protrude. Thus in particular, for some uses, the base plate 1 with only one cutting blade 2 may be sufficient as grinding element, while for some other uses, a grinding element such as grinding element 100 with a larger number of cutting blades 2 may be necessary.

The through cuts have preferably been made using laser cutting or plasma cutting.

The invention has been described by way of specific exemplary embodiments. The scope of the patent has to be construed to encompass not only them but also their legal equivalents.

### List of reference numerals:

R1, R2 distances from bending line
a bend angle
h height of a cutting blade
d base blade thickness
1 base (grinding element)
2 cutting blade
3 beveled face
4 through cut
5 tip
6 beveled face
9 tooth
13 region parallel or essentially parallel with a section of the grinding element adjacent to the surrounding opening
15 pointed tip
20 grinder
100 grinding element
101 jig
102 handle
103 loading spring
104 rod
105 material receiving opening
106 recessed wall portion
107 coding
108 housing
109 preloading knob
120 coding
121 sled

## Claims

1. A grinder (1, 100; 20), especially for preparing micrografts from soft tissue such as cardiac tissue, comprising:
at least one grinding element (1, 100) comprising a number of cutting blades (2), each of which has been made by bending, to an angle (α), a region partially separated from the grinding element (1, 100) by a number of through cuts (4) made in the grinding element (1, 100), whereby the bending has been made at the non-separated part between the cutting blade (2) and the grinding element (1, 100) adjacent to a surrounding opening made by the number of through cuts (4);
**wherein:** each of the number of cutting blades (2) has at least one beveled face (3), and the surrounding opening in the grinding element (1, 100) has respectively at least one beveled face (6) and wherein, through the bending, the at least one beveled face (3) of the cutting blade and the at least one beveled face (6) in the surrounding grinding element (1, 100) are in mutual contact.

2. The grinder (1, 100; 20) according to claim 1, **wherein:** when the grinder is used in grinding, upon a force that will be exerted to a cutting blade (2) by material to be grinded causing a torque trying to bend the blade (2) further and resulting in the beveled faces (3, 6) in mutual contact to cause a counterforce which will be directed towards the grinding element (1, 100) adjacent the surrounding opening, effecting a bending-type tensioning of the cutting blades (2).

3. The grinder (1, 100; 20) according to claim 1 or 2, **wherein:** each of the number of cutting blades (2) has beveled faces (3) at opposite sides, each beveled face (3), through the bending, being in mutual contact with a respective beveled face (6) in the grinding element (1, 100) adjacent the surrounding opening, such that the bending-type tensioning of the cutting blade will be directed to deform the cutting blade (2) into a convex shape.

4. The grinder (1, 100; 20) according to any one of the preceding claims, **wherein:** the through cuts (4) are beveled through cuts having V shape, such that the point of the V shape defines an obtuse angle, a straight angle or an acute angle in the grinding element (1, 100).

5. The grinder (1, 100; 20) of claim 4, **wherein:** the sides of the V shape are beveled such that the beveled edges (3) define at least one acute angle between the surface of the grinding element (1, 100) before bending, in other words such that the at least one, preferably both, of the beveled edges (3) interact with each other and limit the bending of the cutting blade (2) and transfer a torque exerted on it to the grinding element (1, 100) via one or both of the beveled edges (3) to tensioning of the cutting blade (2).

6. The grinder (1, 100; 20) according to claim 4 or 5, **wherein:** the beveled through-cuts (4) intersect such that the beveled edges (3) define the V shape which is part of a pointed tip (15).

7. The grinder (1, 100; 20) according to any one of the preceding claims, **wherein:** the cutting blades (2) are arranged at directional groups each of which has an opposite cutting direction.

8. The grinder (1, 100; 20) according to any one of the preceding claims, **wherein:** some or all of the number of cutting blades (2) have been treated preferably mechanically, such as by grinding, sanding and/or polishing, from the bended side which protrudes from the grinder element (1, 100) such that the cutting blade (2) has a sharpened and/or flattened region (13) from the top that is preferably parallel or essentially parallel with a section of the grinding element (1, 100) adjacent to the surrounding opening.

9. The grinder (1, 100) according to any one of claims 1 to 8, **wherein:** the grinding element (1, 100) is or comprises a cylinder, from which the number of cutting blades (2) protrudes.

10. The grinder (1, 100; 20) according to any one of claims 1 to 8, **wherein:** the grinding element (1, 100) is or comprises a plate-like element, from which the cutting blades (2) protrude.

11. The grinder (20) according to claims 10, **further comprising:** a sled-like sample carrier (121), a handle (102) arranged on the sled (121) such that a movement of the handle (102) moves the sled (121) in relation to the grinding element (1, 100), the sled (121) arranged in a housing (108) that comprises the grinding element (1,100) and limits the movement of the sled (121) however allowing a reciprocal such as back-and-forth motion, and pressing means (104, 103) for pushing material received via a material receiving opening (105) such that the cutting blades (2) will cut the material responsive to the movement of the sled-like sample carrier (121).

12. The grinder (20) according to claim 11, **wherein:** further comprising at least two skids arranged to keep the sled (121) which is located between the skids, in an elevated position with respect to the grinding element (11) such that the elevated position has a clearance (D) which is above the bent cutting blades (2), and wherein, preferably, the housing (108) is configured to limit the movement of the sled (121) such that each of the skids is moveable on the grinding element (1, 100) only at a region (30) that is free of said cutting blades (2).

13. The grinder (20) according to claim 11 or 12, **wherein:** the height of the skids is selected such that the cutting blades (2) do not come into contact with the sled (121).

14. The grinder (20) according to any one of the preceding claims 11 to 13, **wherein:** there is a coding in the sled (121) and/or in the housing (108), defining in which direction the sled (121) is placeable into the grinder (20), allowing the grinding element (1, 100) to be replaced or cleaned.

15. The grinder (1, 100; 20) according to any one of the preceding claims, **wherein:**
the through cuts have been made using laser cutting or plasma cutting;
and/or
the grinding element (1, 100) is made of comprises stainless steel, especially austenitic stainless steel that comprises iron, chromium (between 16-18%), nickel (10-12%) and molybdenum (2-3%), up to 2% manganese, with possibly small (<1%) quantities of silicon, phosphorus and sulfur possibly also being presents;
and/or
the grinding element (1, 100) is made of comprises stainless spring steel, especially SAE 316L grade steel;
and/or
the thickness of the grinding element (1, 100) is between 0,3 mm - 0,6 mm, and advantageously 0,4 mm, especially if the grinding element (1, 100) is made or comprises stainless spring steel such as SAE 316L grade steel.
